(19) Europäisches Patentamt / European Patent Office / Office européen des brevets

(11) **EP 3 779 267 A1**

(12) **EUROPEAN PATENT APPLICATION**
published in accordance with Art. 153(4) EPC

(43) Date of publication:
**17.02.2021 Bulletin 2021/07**

(51) Int Cl.:
*F21S 2/00* (2016.01)   *F21V 23/00* (2015.01)
*F21Y 115/10* (2016.01)   *F21Y 115/30* (2016.01)

(21) Application number: **19785872.3**

(22) Date of filing: **04.03.2019**

(86) International application number:
**PCT/JP2019/008351**

(87) International publication number:
**WO 2019/198380 (17.10.2019 Gazette 2019/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.04.2018   JP 2018076228**

(71) Applicant: **Sony Corporation**
**108-0075 Tokyo (JP)**

(72) Inventors:
• **OKI, Tomoyuki**
  **Tokyo 108-0075 (JP)**
• **NAGASHIMA, Zenya**
  **Tokyo 108-0075 (JP)**

(74) Representative: **D Young & Co LLP**
**120 Holborn**
**London EC1N 2DY (GB)**

(54) **MEDICAL SYSTEM, LIGHT SOURCE DEVICE, AND LIGHT DETECTION METHOD IN LIGHT SOURCE DEVICE**

(57)   To prevent fluctuation in the correlation between the output of a light source and monitor light in a case where the output of the light source is changed.

A medical system according to the present disclosure includes an imaging device that images an observation target, and a light source device that generates light for irradiating the observation target. The light source device has: a multiplexer that multiplexes lights of different colors; a scattering unit that scatters the light multiplexed by the multiplexer; and a light receiving element that detects the light scattered by the scattering unit. By detecting the light scattered by the scattering unit, it is possible to prevent fluctuation in the correlation between the output of the light source and monitor light in a case where the output of the light source is changed.

FIG. 1

**Description**

TECHNICAL FIELD

The present disclosure relates to a medical system, a light source device, and a light detection method of the light source device.

BACKGROUND ART

Conventionally, for example, Patent Document 1 below describes a light source device including: a white light source that emits white light; RGB light sources that can independently adjust the light amount of each emitted light; a multiplexer that multiplexes the white light and the emitted lights to obtain illumination light; and control units that control the light amount of each emitted light.

CITATION LIST

PATENT DOCUMENT

Patent Document 1: International Patent Application Publication No. 2016/092958

SUMMARY OF THE INVENTION

PROBLEMS TO BE SOLVED BY THE INVENTION

In the technology described in the above-mentioned patent document, monitor units for monitoring each of red light, green light, and blue light are provided. However, in a case of a configuration where monitor light is extracted from the optical path before the light is output, if the output of the light source is increased due to the uneven irradiation range of the light, there is a problem that the correlation between the output of the light source and the detection value of the monitor unit fluctuates.

Hence, there has been a need to prevent fluctuation in the correlation between the output of a light source and monitor light in a case where the output of the light source is changed.

SOLUTIONS TO PROBLEMS

According to the present disclosure, provided is a medical system including an imaging device that images an observation target, and a light source device that generates light for irradiating the observation target, in which the light source device has: a multiplexer that multiplexes lights of different colors; a scattering unit that scatters the light multiplexed by the multiplexer; and a light receiving element that detects the light scattered by the scattering unit.

Additionally, according to the present disclosure, provided is a light source device including: a multiplexer that multiplexes lights of different colors; a scattering unit that scatters the light multiplexed by the multiplexer; and a light receiving element that detects the light scattered by the scattering unit.

Additionally, according to the present disclosure, provided is a light detection method of a light source device, the method including: multiplexing lights of different colors; scattering the multiplexed light by a scattering unit; and detecting the light scattered by the scattering unit.

EFFECTS OF THE INVENTION

As described above, according to the present disclosure, it is possible to prevent fluctuation in the correlation between the output of the light source and the monitor light in a case where the output of the light source is changed.

Note that the above-mentioned effects are not necessarily limiting, and any of the effects shown in the present specification or other effects that can be grasped from the present specification can be exerted in addition to or instead of the above-mentioned effects.

BRIEF DESCRIPTION OF DRAWINGS

Fig. 1 is a schematic diagram showing a configuration of a light source device according to an embodiment of the present disclosure.

Fig. 2 is a schematic diagram showing an example of a multiplexing optical system.

Fig. 3 is a schematic diagram showing how RGB light incident on a rod integrator repeats total reflection in the rod integrator.

Fig. 4 is a diagram showing how light leaks from a ridge line of a rectangular rod integrator.

Fig. 5 is a schematic diagram showing some examples of the cross-sectional shape of a rod integrator.

Fig. 6 is a schematic diagram showing a rod integrator and a supporting member that supports the rod integrator.

Fig. 7 is a characteristic diagram showing a correlation between monitor light detected by a light receiving element and device output.

Fig. 8 is a schematic diagram showing a comparative example in which a light receiving element is arranged at a reflected position from a condenser lens to acquire monitor light.

Fig. 9 is a characteristic diagram showing a correlation between the monitor light output and the device output obtained in the configuration example shown in Fig. 8.

Fig. 10 is a schematic diagram showing a configuration example in which a housing is used as a scattering optical system.

Fig. 11 is a schematic diagram showing a configuration of a medical system including a light source device.

MODE FOR CARRYING OUT THE INVENTION

Hereinafter, preferred embodiments of the present disclosure will be described in detail with reference to the accompanying drawings. Note that in the present specification and the drawings, components having substantially the same functional configuration will be assigned the same reference numerals to omit redundant description.

Note that the description will be given in the following order.

1. Prerequisite technology
2. Overall configuration example of light source device
3. Configuration example of multiplexing optical system
4. Configuration example of rod integrator
5. Configuration example of scattering optical system
6. Correlation between monitor light and device output
7. Configuration example using housing as scattering optical system
8. Configuration example of medical system

1. Prerequisite technology

An endoscope is widely used as a device for observing the internal structure of an object. Endoscopes have rapidly spread particularly in the medical field with the development of operative technologies, and have become indispensable in many healthcare fields.

In a conventional endoscope apparatus, a single light source such as a lamp light source (xenon lamp or halogen lamp) or a white LED light source is mainly used as an illuminating light source regardless of whether it is a flexible endoscope or a rigid endoscope.

On the other hand, since white light can be generated by combining multiple lights, a light source that multiplexes a red LED, a green LED, and a blue LED has also been produced, for example. Such a light source has an advantage that the hue of white can be freely set, but on the other hand, accurate control of the light distribution ratio is required. In order to control the distribution ratio, it is necessary to accurately grasp the output level of the light source currently emitting light. Even in a case where the distribution ratio is slightly inappropriate, the observer's eyes will see light, which should originally be white, with color such as in "reddish white". For this reason, accurate control of the distribution ratio as described above is required.

On the other hand, in order to grasp the output of a light source, there is a method of extracting monitor light that is correlated with device output of the light source device from the optical path before the light is output, and grasping the output of the light source by fluctuation of the monitor light.

In general, a semiconductor light source such as a light emitting diode (LED) or a laser diode (LD) tends to have a wider radiation angle when the output is increased (when applied current is increased). Additionally, the intensity of the emitted light varies within the range of the radiation angle. For this reason, when the output of the light source is increased, light incident on a light receiving element fluctuates due to this variation as the radiation angle spreads. This tendency becomes more prominent as the light receiving area of the light receiving element becomes smaller relative to the radiation angle range. Accordingly, the method of extracting the monitor light from the optical path cannot improve the correlation between the output of the light source and the output of the monitor light.

In view of the above points, in the present embodiment, monitor light that is not easily affected by the unevenness of the intensity of light generated within the range of the radiation angle is received by a light receiving element, and the value is fed back to a drive unit to control the amount of illumination light.

2. Overall configuration example of light source device

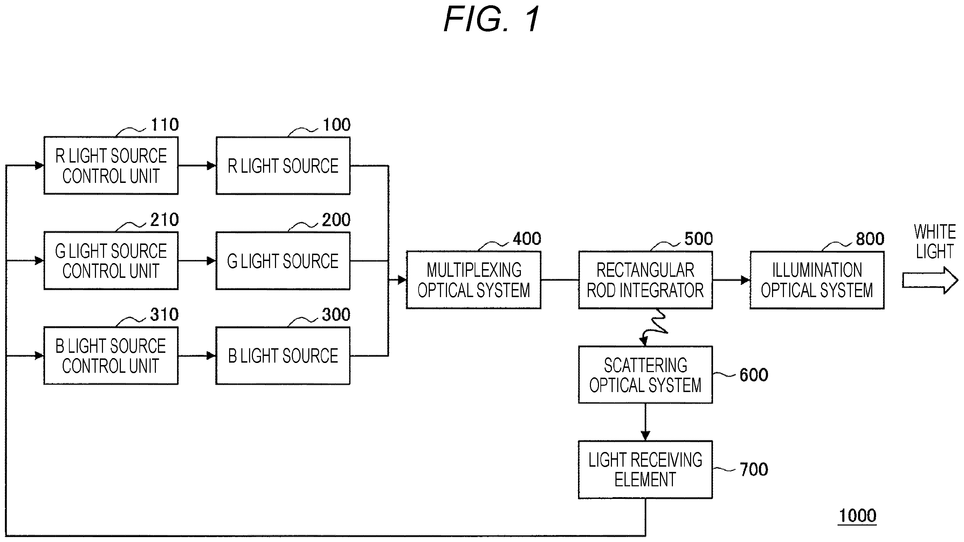

First, a configuration of a light source device 1000 according to an embodiment of the present disclosure will be described with reference to Fig. 1. As shown in Fig. 1, the light source device 1000 includes a red light source (R light source) 100, a green light source (G light source) 200, a blue light source (B light source) 300, a red light source control unit 110, a green light source control unit 210, a blue light source control unit 310, a multiplexing optical system (multiplexer) 400, a rectangular rod integrator (optical member) 500, a scattering optical system (scattering unit) 600, a light receiving element 700, and an illumination optical system 800.

Each of the red light source 100, the green light source 200, and the blue light source 300 includes a semiconductor laser. For example, a GaInP quantum well structure laser diode (RLD) is used as the red light source 100, a GaInN quantum well structure laser diode (GLD) is used as the green light source 200, and a GaInN quantum well structure laser diode (BLD) is used as the blue light source 300.

The red light source control unit 110, the green light source control unit 210, and the blue light source control unit 310 control the red light source 100, the green light source 200, and the blue light source 300, respectively. The multiplexing optical system 400 combines the lights emitted from the red light source 100, the green light source 200, and the blue light source 300.

The light receiving element 700 includes an RGB color sensor that can detect the visible light region by dividing it into red, green, and blue color signals. More specifically, the light receiving element 700 includes a photodiode combined with an RGB color filter. The illumination optical system 800 is an optical system for irradiating an observation target with white light obtained from multiplexing by the multiplexing optical system 400.

3. Configuration example of multiplexing optical system

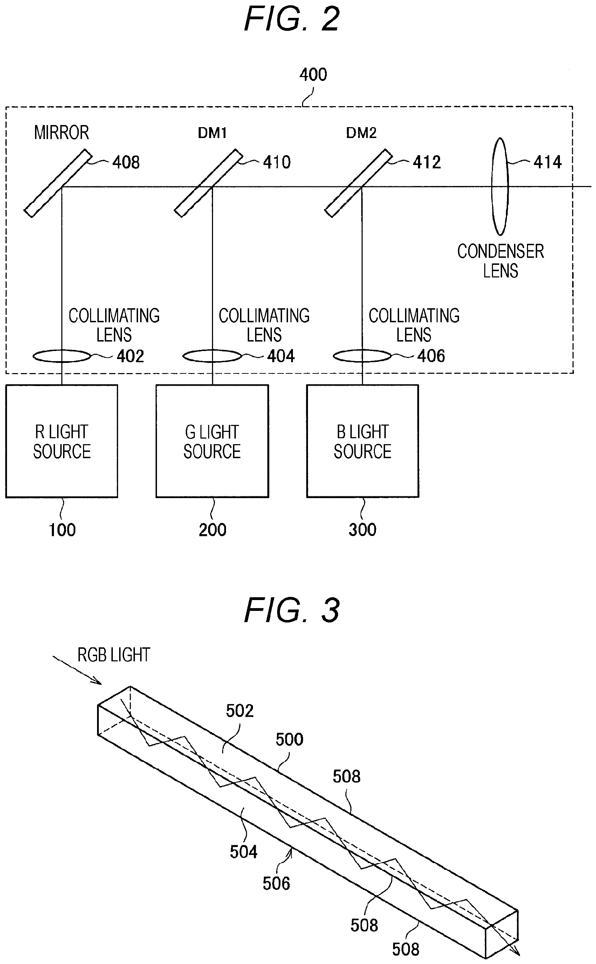

Fig. 2 is a schematic diagram showing an example of the multiplexing optical system 400. As shown in Fig. 2, the multiplexing optical system 400 includes collimating lenses 402, 404, 406, a mirror 408, a dichroic mirror (DM1) 410, a dichroic mirror (DM2) 412, and a condenser lens 414.

Light rays emitted from the red light source 100, the green light source 200, and the blue light source 300 are respectively collimated by the collimating lenses 402, 404, and 406. The red light is reflected by the mirror 408 at an angle of 90 degrees, passes through the dichroic mirror 410 and the dichroic mirror 412, and is condensed by the condenser lens 414. The green light is emitted toward the dichroic mirror 410, and the blue light is emitted toward the dichroic mirror 412.

The dichroic mirror 410 has an optical characteristic of transmitting a red wavelength and reflecting a green wavelength. The dichroic mirror 412 has an optical characteristic of transmitting a red wavelength and a green wavelength and reflecting a blue wavelength. The red wavelength from the red light source 100 is multiplexed with the green wavelength from the green light source 200 by the dichroic mirror 410, and is multiplexed with the blue wavelength from the blue light source 300 by the dichroic mirror 412. The multiplexed light is condensed by the condenser lens 414. As described above, by multiplexing the red wavelength, the green wavelength, and the blue wavelength, a white light laser can be emitted from the multiplexing optical system 400.

RGB light condensed by the common condenser lens 414 is coupled to the rod integrator 500. The rod integrator 500 includes a transparent glass material, for example, and has a prismatic shape. The rod integrator 500 has a rectangular (square) cross section in a direction orthogonal to the light traveling direction. The RGB light incident on the rod integrator 500 is repeatedly totally reflected within the rod integrator 500, and is emitted from an end face on the opposite side of an end face on the incident side.

4. Configuration example of rod integrator

Fig. 3 is a schematic diagram showing how RGB light incident on the rod integrator 500 repeats total reflection inside the rod integrator 500. The RGB light is totally reflected by planes such as an upper surface 502, a side surface 504, and a lower surface 506 of the rod integrator 500, and proceeds through the rod integrator 500 while repeating total reflection as shown by an arrow in Fig. 3. Thus, the RGB light is basically confined inside the rod integrator 500 by total reflection and travels through the rod integrator 500.

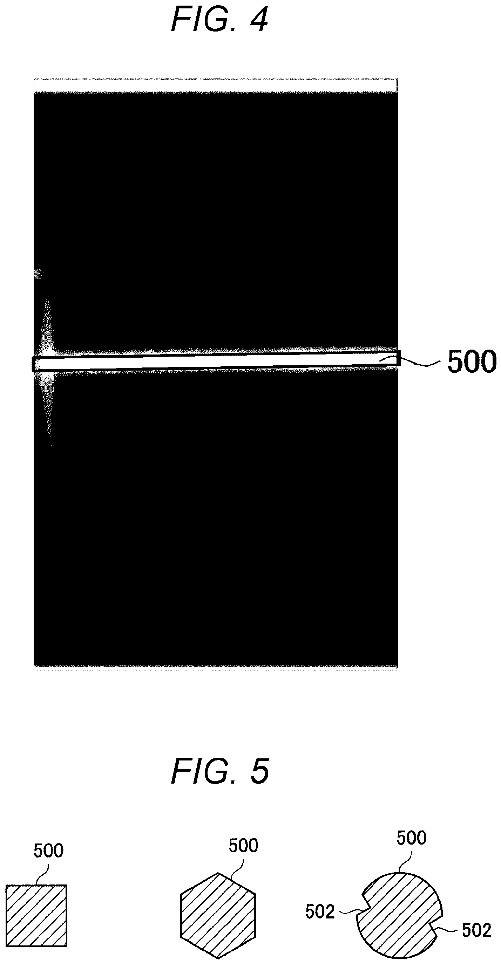

On the other hand, in the vicinity of a ridge line (edge) 508 of the rod integrator 500, the RGB light leaks out of the rod integrator 500. If the rod integrator 500 has a round cross-section, there is no light leakage. However, if the rod integrator 500 has a polygonal cross-section with the ridge line 508, light will be mixed at the ridge line 508, and a slight amount of light leaks from the ridge line 508. Fig. 4 is a diagram showing how light leaks from the ridge line 508 of the rectangular rod integrator 500. As shown in Fig. 4, it can be seen that light leaks around the contour of the outer shape of the rod integrator 500. In the present embodiment, the leaked light is received by the light receiving element 700 as monitor light, and feedback is provided to the red light source control unit 110, the green light source control unit 210, and the blue light source control unit 310 which are control systems of the light sources, the red light source 100, the green light source 200, and the blue light source 300, respectively. As described above, the light receiving element 700 can detect the visible light region by dividing it into red, green, and blue color signals. Hence, the light receiving element 700 can control the red light source 100, the green light source 200, and the blue light source 300 on the basis of the monitor light to optimally control each light source, and can perform control, so that the light multiplexed by the multiplexing optical system 400 is white light.

Accordingly, by making the cross-sectional shape of the rod integrator 500 polygonal and providing the ridge lines, leaked light can be generated, and the leaked light can be detected as monitor light. Here, the sharper the edge of the ridge line, the larger the amount of leaked light, and leaked light with higher intensity can be detected. Accordingly, in a case where the rod integrator 500 has a triangular cross-sectional shape, the intensity of leaked light can be maximized. On the other hand, in a case of using the RGB light as the light source of an endoscope, for example, the light of the cross-sectional shape of the rod integrator 500 is finally emitted to the affected area. Hence, it is possible to illuminate a wider area and visibility is improved by emitting light of a shape close to a circle than emitting light of a triangular shape. Accordingly, in such a case, it is preferable that the cross-sectional shape of the rod integrator 500 is a square or a polygon having more vertices than a square.

Additionally, when the intensity of the leaked light is low, gain is increased to increase the output of the monitor light, and the influence of noise becomes large. For this reason, the influence of noise can be reduced by increasing the intensity of leaked light and detecting the monitor light.

Accordingly, the cross-sectional shape of the rod integrator 500 can be appropriately set according to the environment in which the light source device 1000 is used and the required intensity of leaked light.

Additionally, by making the cross section of the rod integrator 500 a polygonal shape, RGB light proceeds while repeating total reflection in the rod integrator 500. Hence, it is possible to reliably eliminate the unevenness of red, green, and blue due to the mixing effect.

Fig. 5 is a schematic diagram showing some examples of the cross-sectional shape of the rod integrator 500. As shown in Fig. 5, a polygonal shape such as a square or a hexagon can be used as the cross-sectional shape of the rod integrator 500. Note that the number of vertices of the polygonal shape may be three or more. Additionally, as shown in the diagram on the right side of Fig. 5, the rod integrator 500 may have a circular cross-sectional shape and notches 502 may be provided. In this case, leaked light can be generated at the notches 502.

5. Configuration example of scattering optical system

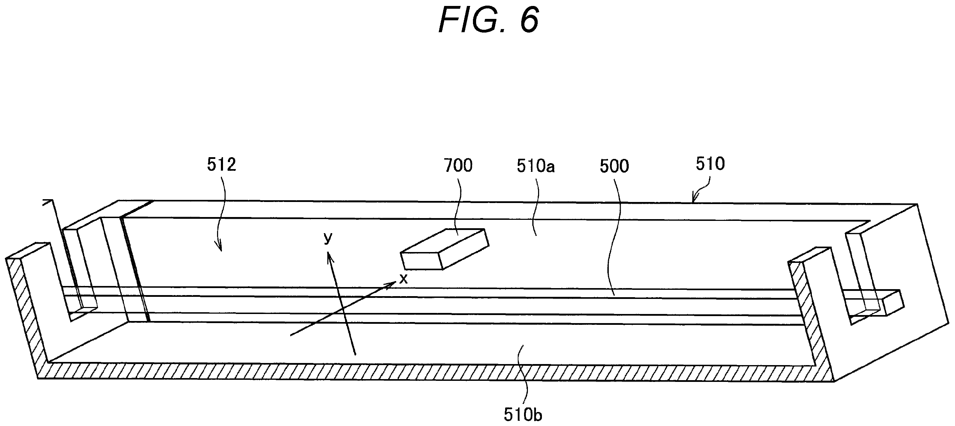

Fig. 6 is a schematic diagram showing a rod integrator 500 and a supporting member 510 that supports the rod integrator 500. The supporting member 510 corresponds to the scattering optical system 600 shown in Fig. 1. As shown in Fig. 6, the vicinities of both end portions of the rod integrator 500 are supported by the supporting member 510. The supporting member 510 includes a metal such as aluminum, for example, and reflects light on its surface. The supporting member 510 is provided with a recess 512 along the extending direction of the rod integrator 500, and the rod integrator 500 is disposed in the recess 512. As a result, the rod integrator 500 is surrounded by both side surfaces and a lower surface 510b of the recess 512. Note that Fig. 6 shows a state in which the supporting member 510 is cut along a hatched cut surface. Hence, in Fig. 6, only one side surface 510a of both side surfaces of the recess 512 located on both sides of the rod integrator 500 is shown, and the side surface opposite to the side surface 510a is not shown.

With such a configuration, the leaked light from the ridge lines of the rod integrator 500 is scattered and reflected by both side surfaces and the lower surface 510b of the recess 510. In other words, in the coordinate system shown in Fig. 6, among the wall surfaces included in the recess 512, the leaked light is scattered and reflected on three surfaces of the side surface 510a located in the +x direction, the side surface (not shown) located in the -x direction, and the lower surface 510b located in the -y direction.

Then, the recess 512 is open in the +y direction, and the light receiving element 700 is arranged therein. That is, the light receiving element 700 is arranged at a position facing the lower surface 510b of the recess 512 with the rod integrator 500 interposed therebetween. The light receiving surface of the light receiving element 700 is directed toward the rod integrator 500.

With this arrangement, the leaked light from the four ridge lines 508 of the rod integrator 500 is scattered in the recess 512, and the scattered leaked light is detected by the light receiving element 700. By scattering the leaked light, changes due to the excited state of the light source are reduced, and stable monitor light can be detected. Accordingly, by controlling the red light source 100, the green light source 200, and the blue light source 300 on the basis of such monitor light, it is possible to provide illumination light with a stable color temperature.

Note that while the upper portion of the recess 512 is open in the example shown in Fig. 6, the upper portion of the recess 512 may be closed so that the leaked light is scattered and reflected also on an upper surface of the rod integrator 500.

The light receiving surface of the light receiving element 700 is arranged so as to be parallel to the extending direction of the rod integrator 700. Note that even if the light receiving surface of the light receiving element 700 is inclined with respect to the extending direction of the rod integrator 700, if the positional relationship between the light receiving surface and the rod integrator 700 and supporting member 510 is fixed, the monitor light can be detected stably. Accordingly, the light receiving surface of the light receiving element 700 only needs to be directed toward the rod integrator 500.

Additionally, the intensity of the leaked light is higher on the emission side of the light from the rod integrator 500 than on the incidence side of the light to the rod integrator 500. Accordingly, by arranging the light receiving element 700 at a position closer to the end face on the light emission side in the longitudinal direction of the rod integrator 500, it is possible to increase the light reception intensity of leaked light.

As described above, although it is possible to detect the leaked light by the light receiving element 700 without scattering the leaked light, by scattering the leaked light by the scattering optical system 600, it is possible to detect more stable monitor light.

6. Correlation between monitor light and device output

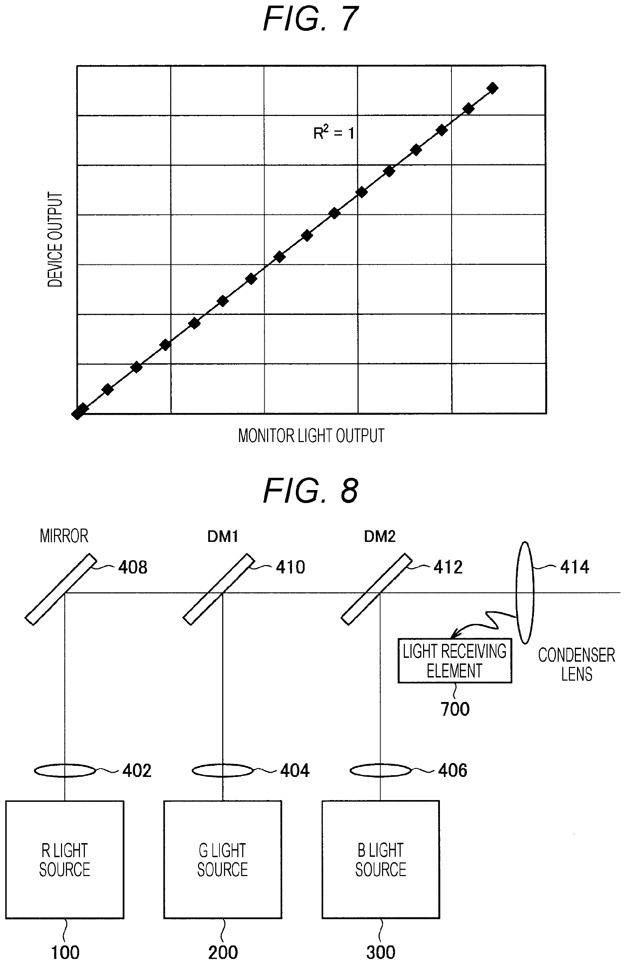

Fig. 7 is a characteristic diagram showing a correlation between monitor light detected by the light receiving element 700 and device output of the light source device 1000. The device output of the light source device 1000 indicates the intensity of light emitted by the light source device 1000. Additionally, output of the monitor light indicates the intensity of the monitor light detected by the light receiving element 700. As shown in Fig. 7, it can be seen that the output of the monitor light and the device output of the light source device 1000 have an excellent linear relationship.

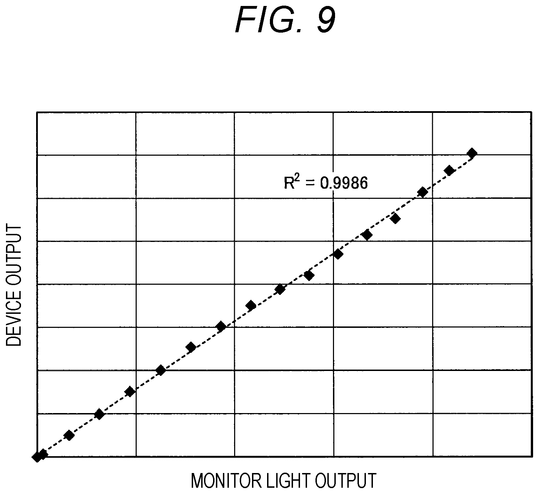

On the other hand, Fig. 8 is a schematic diagram showing a comparative example in which a light receiving element 700 is arranged at a reflected position from the condenser lens 414 to acquire monitor light. Additionally, Fig. 9 is a characteristic diagram showing a correlation between the output of the monitor light and the device output obtained in the configuration example shown in Fig. 8. Although the configuration example as shown in Fig. 8 has been conventionally proposed, the linear relationship between the monitor light output and the device output is destroyed as shown in Fig. 9. This phenomenon is caused by the aforementioned phenomenon that the radiation angle increases as the output of the semiconductor laser increases, and the correlation is destroyed depending on the position of the light receiving element and the light receiving area. Note that $R^2$ shown in Figs. 7 and 9 represents the square value of a correlation coefficient (r). The value of $R^2$ is larger and closer to one in Fig. 7, which indicates that the correlation between the output of the monitor light and the device output is good. If there are n sets of data for variables x and y (($x_1$, $y_1$), ($x_2$, $y_2$),..., ($x_n$, $y_n$)), the correlation coefficient (r) between them is given by the following equation.

[Expression 1]

$$r_{xy} = \frac{\sum_{i=1}^{n} (x_i - \bar{x})(y_i - \bar{y})}{\sqrt{\sum_{i=1}^{n} (x_i - \bar{x})^2} \sqrt{\sum_{i=1}^{n} (y_i - \bar{y})^2}}$$

Here, $\bar{x}$ and $\bar{y}$ each represent the mean value.

Hence, in the configuration example shown in Fig. 8, it is not possible to obtain a good correlation between the monitor light output and the device output. Accordingly, it is difficult to accurately control the red light source 100, the green light source 200, and the blue light source 300 on the basis of the monitor light output detected by the light receiving element 700, and obtain white light.

As described above, according to the present embodiment, since the leaked light from the rod integrator 500 is scattered and reflected by the recess 512 of the supporting member 510 and detected by the light receiving element 700, a good correlation between the monitor light output and the device output can be obtained. Accordingly, the red light source 100, the green light source 200, and the blue light source 300 can be accurately controlled on the basis of the monitor light.

7. Configuration example using housing as scattering optical system

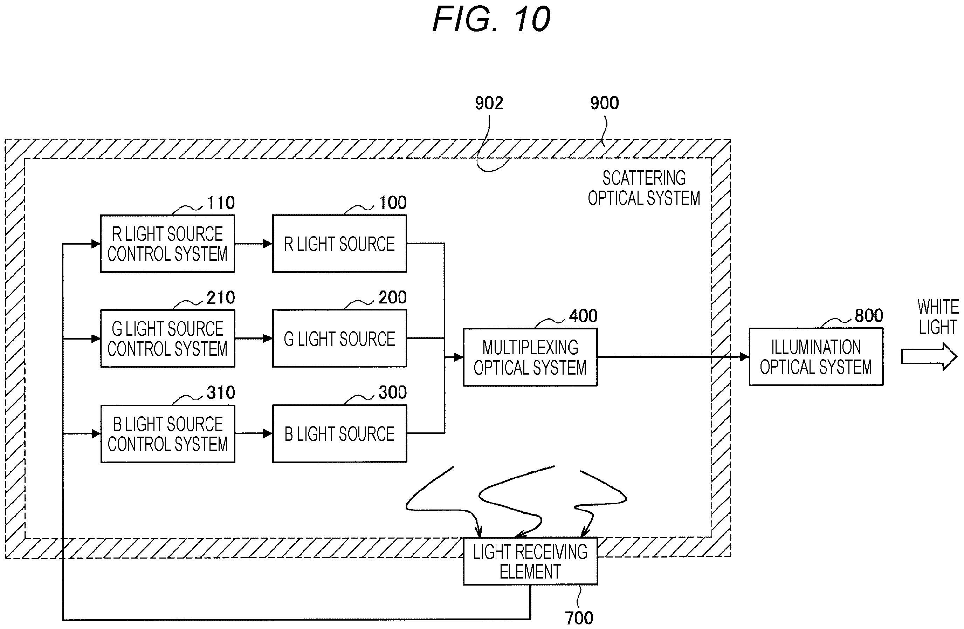

In the above-mentioned example, in order to use the scattered and reflected light as the monitor light, the leaked light from the ridge lines of the rod integrator 500 is used. Meanwhile, Fig. 10 is a schematic diagram showing a configuration example in which a housing is used as a scattering optical system 600. As shown in Fig. 10, components including a red light source 100, a green light source 200, a blue light source 300, and a multiplexing optical system 400 are sealed in a housing 900, and light mixed by scattering due to reflection on an inner surface 902 of the housing 900 is monitored.
In this case, the inner surface 902 of the housing 900 can be non-coated having no surface treatment. By including a metal such as aluminum in the housing 900 and making the inner surface 902 non-coated as similar to the supporting member 510 described above, leaked light from the red light source 100, the green light source 200, and the blue light source 300, or from the multiplexing optical system 400 can be scattered and reflected by the inner surface 902.
Additionally, the inner surface 902 of the housing 900 may be subjected to a blasting treatment to roughen the surface, or be subjected to a surface treatment such as white alumite treatment or barium sulfate coat treatment, which is more likely to cause scattering and reflection. As a result, the leaked light from the red light source 100, the green light source 200, and the blue light source 300, or from the multiplexing optical system 400 can be well scattered and reflected on the inner surface 902. Thus, the inner surface 902 of the housing 900 can be formed in any way as long as it does not absorb leaked light.
As shown in Fig. 10, a light receiving element 700 is provided in the housing 900. The light receiving surface of the light receiving element 700 is directed toward the inside of the housing 900. With this arrangement, the leaked light scattered in the housing 900 can be detected by the light receiving element 700 as monitor light.

8. Configuration example of medical system

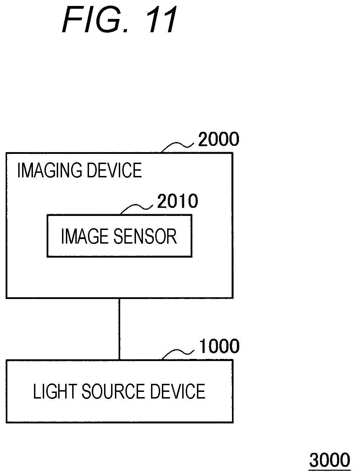

Fig. 11 is a schematic diagram showing a configuration of a medical system 3000 including a light source device 1000. The medical system 3000 shown in Fig. 11 is a system such as an endoscope system and a microscope system. As shown in Fig. 11, the medical system 3000 includes an imaging device (camera head) 2000 in addition to the light source device 1000.
An optical system and an image sensor 2010 are provided inside the imaging device 2000. Reflected light (observation light) from the observation target is condensed on the image sensor 2010 by the optical system. The observation light is photoelectrically converted by the image sensor 2010, and an electrical signal corresponding to the observation light, that is, an image signal corresponding to the observed image is generated.
Light generated by the light source device 1000 is emitted toward the observation target. In the case of an endoscope system, light generated by the light source device 1000 is guided to the tip end of a lens barrel of an endoscope connected to the imaging device 2000 by a light guide extending inside the lens barrel, and is emitted toward the observation target in the body cavity of the patient. Additionally, in the case of the microscope system, light generated by the light source device 1000 is directly emitted from the imaging device 2000 toward the observation target. Additionally, light generated by the light source device 1000 may be directly emitted to the observation target without passing through the imaging device 2000.
As described above, according to the present embodiment, leaked light from the red light source 100, the green light source 200, and the blue light source 300 is scattered, and the scattered light is detected by the light receiving element 700 as monitor light. With this configuration, it possible to obtain a good correlation between the output of the light source device and the monitor light. Accordingly, it becomes possible to optimally control the light source device on the basis of the monitor light output.
Hereinabove, preferred embodiments of the present disclosure have been described in detail with reference to the accompanying drawings. However, the technical scope of the present disclosure is not limited to such examples. It will be apparent to those skilled in the art of the present disclosure that various change examples or modification examples can be conceived within the scope of the technical idea described in the claims. It is understood that these also belong to the technical scope of the present disclosure, as a matter of course.
For example, while the above embodiment shows an example in which the present technology is applied to a light source device mainly used for medical purposes, the present technology is not limited to such an example. For example, the present technology may be applied to a light source device such as a projector. Additionally, the present technology may be applied to a light source device for industrial use such as an industrial endoscope.
Additionally, the effects described in the present specification are merely illustrative or exemplary, and are not limiting. That is, the technology according to the present disclosure can exhibit other effects apparent to those skilled in the art from the description of the present specification, in addition to or instead of the effects described above.
Note that the following configurations are also within the technical scope of the present disclosure.

(1) A medical system including:

an imaging device that images an observation target; and
a light source device that generates light for irradiating the observation target, in which
the light source device has:

a multiplexer that multiplexes lights of different colors;
a scattering unit that scatters the light multiplexed by the multiplexer; and
a light receiving element that detects the light scattered by the scattering unit.

(2) A light source device including:

a multiplexer that multiplexes lights of different colors;
a scattering unit that scatters the light multiplexed by the multiplexer; and
a light receiving element that detects the light scattered by the scattering unit.

(3) The light source device according to (2) above further including
an optical member through which the light multiplexed by the multiplexer is transmitted, in which
the scattering unit scatters leaked light leaking from the optical member.
(4) The light source device according to (3) above, in which the optical member has a ridge line extending in a light traveling direction on an outer surface thereof.
(5) The light source device according to (4) above, in which the leaked light is leaked light from the ridge line.
(6) The light source device according to (3) above, in which the optical member has a polygonal cross-sectional shape orthogonal to a light traveling direction.
(7) The light source device according to any one of (3) to (6) above, in which the scattering unit is arranged so as to surround a side surface of the optical member extending along a light traveling direction.
(8) The light source device according to (7) above, in which the scattering unit includes a groove-shaped recess formed so as to surround the side surface of the optical member.
(9) The light source device according to any one of (3) to (8) above, in which a light receiving surface of the light receiving element is directed toward the optical member.
(10) The light source device according to any one of (2) to (9) above further including:

a red light source that generates red light;
a green light source that generates green light; and
a blue light source that generates blue light, in which
white light is obtained by multiplexing the red light, the green light, and the blue light by the multiplexer.

(11) The light source device according to (10) above further including a light source control unit that controls the red light source, the green light source, or the blue light source on the basis of an optical signal detected by the light receiving element.
(12) The light source device according to (10) or (11) above, in which the scattering unit is a housing that stores the red light source, the green light source, and the blue light source.
(13) The light source device according to (12) above, in which an inner surface of the housing is subjected to a blasting treatment.
(14) The light source device according to (12) above, in which an inner surface of the housing is subjected to white alumite treatment or barium sulfate coat treatment.
(15) The light source device according to (12) above, in which the housing includes a metal, and an inner surface of the housing is not subjected to any surface treatment.
(16) A light detection method of a light source device, the method including:

multiplexing lights of different colors;
scattering the multiplexed light by a scattering unit; and detecting the light scattered by the scattering unit.

REFERENCE SIGNS LIST

100    Red light source
110    Red light source control unit
200    Green light source

| | |
|---|---|
| 210 | Green light source control unit |
| 300 | Blue light source |
| 310 | Blue light source control unit |
| 400 | Multiplexing optical system |
| 500 | Rod integrator |
| 508 | Ridge line |
| 510 | Supporting member |
| 512 | Recess |
| 600 | Scattering optical system |
| 700 | Light receiving element |
| 900 | Housing |
| 902 | Inner surface |
| 1000 | Light source device |
| 2000 | Imaging device |
| 3000 | Medical system |

**Claims**

1. A medical system comprising:

   an imaging device that images an observation target; and
   a light source device that generates light for irradiating the observation target, wherein
   the light source device has:

      a multiplexer that multiplexes lights of different colors;
      a scattering unit that scatters the light multiplexed by the multiplexer; and
      a light receiving element that detects the light scattered by the scattering unit.

2. A light source device comprising:

   a multiplexer that multiplexes lights of different colors;
   a scattering unit that scatters the light multiplexed by the multiplexer; and
   a light receiving element that detects the light scattered by the scattering unit.

3. The light source device according to claim 2 further comprising
   an optical member through which the light multiplexed by the multiplexer is transmitted, wherein
   the scattering unit scatters leaked light leaking from the optical member.

4. The light source device according to claim 3,
   wherein the optical member has a ridge line extending in a light traveling direction on an outer surface thereof.

5. The light source device according to claim 4,
   wherein the leaked light is leaked light from the ridge line.

6. The light source device according to claim 3,
   wherein the optical member has a polygonal cross-sectional shape orthogonal to a light traveling direction.

7. The light source device according to claim 3,
   wherein the scattering unit is arranged so as to surround a side surface of the optical member extending along a light traveling direction.

8. The light source device according to claim 7,
   wherein the scattering unit includes a groove-shaped recess formed so as to surround the side surface of the optical member.

9. The light source device according to claim 3,
   wherein a light receiving surface of the light receiving element is directed toward the optical member.

**10.** The light source device according to claim 2 further comprising:

a red light source that generates red light;
a green light source that generates green light; and
a blue light source that generates blue light, wherein
white light is obtained by multiplexing the red light, the green light, and the blue light by the multiplexer.

**11.** The light source device according to claim 10 further comprising a light source control unit that controls the red light source, the green light source, or the blue light source on a basis of an optical signal detected by the light receiving element.

**12.** The light source device according to claim 10,
wherein the scattering unit is a housing that stores the red light source, the green light source, and the blue light source.

**13.** The light source device according to claim 12,
wherein an inner surface of the housing is subjected to a blasting treatment.

**14.** The light source device according to claim 12,
wherein an inner surface of the housing is subjected to white alumite treatment or barium sulfate coat treatment.

**15.** The light source device according to claim 12,
wherein the housing includes a metal, and an inner surface of the housing is not subjected to any surface treatment.

**16.** A light detection method of a light source device, the method comprising:

multiplexing lights of different colors;
scattering the multiplexed light by a scattering unit; and
detecting the light scattered by the scattering unit.

## FIG. 1

- R LIGHT SOURCE CONTROL UNIT — 110
- R LIGHT SOURCE — 100
- G LIGHT SOURCE CONTROL UNIT — 210
- G LIGHT SOURCE — 200
- B LIGHT SOURCE CONTROL UNIT — 310
- B LIGHT SOURCE — 300
- MULTIPLEXING OPTICAL SYSTEM — 400
- RECTANGULAR ROD INTEGRATOR — 500
- ILLUMINATION OPTICAL SYSTEM — 800
- WHITE LIGHT
- SCATTERING OPTICAL SYSTEM — 600
- LIGHT RECEIVING ELEMENT — 700
- 1000

# FIG. 2

400

MIRROR       DM1       DM2

408      410      412      414

CONDENSER
LENS

COLLIMATING
LENS    402

COLLIMATING
LENS    404

COLLIMATING
LENS    406

R LIGHT
SOURCE

G LIGHT
SOURCE

B LIGHT
SOURCE

100       200       300

# FIG. 3

RGB LIGHT

502
500
508
504
506
508
508

## FIG. 4

500

## FIG. 5

500

500

500

502

502

# FIG. 6

# FIG. 7

$R^2 = 1$

DEVICE OUTPUT

MONITOR LIGHT OUTPUT

# FIG. 8

MIRROR 408  DM1 410  DM2 412  414

LIGHT RECEIVING ELEMENT 700  CONDENSER LENS

402  404  406

R LIGHT SOURCE  G LIGHT SOURCE  B LIGHT SOURCE

100  200  300

# FIG. 9

R² = 0.9986

DEVICE OUTPUT

MONITOR LIGHT OUTPUT

## FIG. 10

EP 3 779 267 A1

# FIG. 11

IMAGING DEVICE 2000

IMAGE SENSOR 2010

LIGHT SOURCE DEVICE 1000

3000

## INTERNATIONAL SEARCH REPORT

International application No.

PCT/JP2019/008351

### A. CLASSIFICATION OF SUBJECT MATTER

Int.Cl. F21S2/00(2016.01)i, F21V23/00(2015.01)i, F21Y115/10(2016.01)n, F21Y115/30(2016.01)n

According to International Patent Classification (IPC) or to both national classification and IPC

### B. FIELDS SEARCHED

Minimum documentation searched (classification system followed by classification symbols)
Int.Cl. F21S2/00, F21V23/00, F21Y115/10, F21Y115/30

Documentation searched other than minimum documentation to the extent that such documents are included in the fields searched
Published examined utility model applications of Japan   1922–1996
Published unexamined utility model applications of Japan   1971–2019
Registered utility model specifications of Japan   1996–2019
Published registered utility model applications of Japan   1994–2019

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y<br>A | JP 2015-210954 A (OLYMPUS CORPORATION) 24 November 2015, paragraphs [0017]-[0022], [0025], [0026], [0029], [0050], fig. 1-3, 7<br>& US 2017/0038514 A1, paragraphs [0029]-[0033], [0036], [0037], [0040], [0062], fig. 1-3, 7 | 1-11, 16<br>12-15 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02.04.2019 | 16.04.2019 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
|---|
| PCT/JP2019/008351 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2013/146014 A1 (OLYMPUS MEDICAL SYSTEMS CORPORATION) 03 October 2013, paragraphs [0021], [0023], [0026]-[0029], [0033], [0036], [0037], [0039], [0060], fig. 1 & US 2014/0049624 A1, paragraphs [0030], [0032], [0035]-[0038], [0042], [0045], [0046], [0048], [0069], fig. 1 & EP 2737843 A1 & CN 103796571 A | 1-11, 16 |
| Y | JP 2014-148447 A (MITSUBISHI CABLE IND LTD.) 21 August 2014, paragraph [0024] (Family: none) | 6 |
| Y | JP 2000-260559 A (SONY CORPORATION) 22 September 2000, paragraph [0038] & US 2002/0122642 A1, paragraph [0054] | 6 |
| A | JP 2002-368321 A (SUMITOMO ELECTRIC INDUSTRIES, LTD.) 20 December 2002, entire text, all drawings (Family: none) | 1-16 |
| A | JP 2011-44379 A (HARISON TOSHIBA LIGHTING CORPORATION) 03 March 2011, entire text, all drawings (Family: none) | 1-16 |

Form PCT/ISA/210 (continuation of second sheet) (January 2015)

## REFERENCES CITED IN THE DESCRIPTION

**Patent documents cited in the description**

- WO 2016092958 A